# EUROPEAN PATENT SPECIFICATION

(11) **EP 2 566 527 B1**
(45) Date of publication and mention of the grant of the patent: **30.11.2016**
(21) Application number: 11716580.3
(22) Date of filing: 02.05.2011
(51) Int. Cl.: A61L 27/22

(54) **MEDICAL HOLLOW BODY IMPLANT**
MEDIZINISCHES HOHLKÖRPERIMPLANTAT
IMPLANT MÉDICAL À CORPS CREUX

(30) Priority: 05.05.2010 DE 102010020663
(43) Date of publication of application: 13.03.2013
(73) Proprietor: Aesculap AG, 78532 Tuttlingen (DE)
(72) Inventor: BLENDER, Bernd, 78532 Tuttlingen (DE); ODERMATT, Erich, 8200 Schaffhausen (CH); WEGMANN, Jürgen, 78333 Stockach (DE)
(74) Representative: Patentanwälte Ruff, Wilhelm, Beier, Dauster & Partner mbB
(86) International application number: PCT/EP2011/056917
(87) International publication number: WO 2011/138258

(56) References cited:
- WO-A1-2010/006270
- US-A- 5 350 583
- US-A1- 2003 133 967
- US-A1- 2003 171 824
- US-A1- 2006 136 047

## Description

The invention relates to a process for producing a medical hollow body implant, more particularly a tubular or tube-shaped implant, and also to a medical implant obtained or obtainable by the process.

US 2005/0175659 A1 discloses crosslinked plates of collagen which are prepared from collagen suspensions by lyophilisation and subsequent crosslinking. The collagen plates produced have a porous structure and hence the collagen plates have only limited utility for medical applications requiring enhanced dimensional stability and/or imperviousness to liquids.

US 2006/0136047 A1 discloses multiply laminated tubes whose lumens are lined with a layer of juvenile submucosa. The juvenile submucosa is recovered from natural intestines. To produce the tubes, the submucosa is grown on a carrier rod. For this, the submucosa can already be provided in its natural tubular form. To construct the tubes, further layers are applied to the carrier rod which are interconnectable using fibrin adhesive or a crosslinking step for example. However, the recovery of natural intestinal submucosa makes the production of the tubes comparatively costly and complicated. Owing to the naturally predetermined diameter of juvenile intestines, moreover, it is not possible to realize just any desired tubular diameter.

US 6,334,872 B1 describes two- or three-ply collagen tubes, the individual plies of which have different properties and are made of different collagens. The inner ply of the collagen tubes is made of acid-extracted collagen for instance. The inner ply also has a smooth surface, resulting in a reduced risk of thrombosis. The outer ply is recovered from intestinal tissue, fascia lata or dura mater of a mammal, and is primarily responsible for the stability of the tubes. The tube shape is produced using suture, stapler or fibrin adhesive. However, these fixing elements required to produce the tubular shape represent an increased introduction of foreign material into the body of a patient. And the production process is made complicated and costly as a result.

US 7,615,063 B2 describes tubular collagen structures for nerve track regeneration. The lumen of the tubular structures is endowed with a sponge material which contains channels for nerve track regeneration. The tube shape is produced by extrusion or spinning of an aqueous collagen solution onto a carrier rod and hence again by a comparatively complicated technology.

US 7,338,517 reveals a collagen tube, the collagen fibres or fibrils of which are aligned in a helical pattern along the longitudinal axis of the tube. Production is by extrusion of a collagen dispersion in a counterrotating extruder. The helical alignment of the fibres or fibrils is defined by the speed of movement of the receiving yarn roller and also the speed of extrusion of the collagen dispersion. The process is likewise comparatively complicated and costly. Another disadvantage is that the tubes obtained have but limited stability in storage. To maintain their tubular structure, the tubes are exposed to a gas stream while they are immersed in a water bath.

US 6,589,257 B1 discloses a tube constructed with three layers which is usable as an artificial nerve guide track in particular. The inner and outer layers are made of collagen or gelatine. The middle layer consists of a net of an absorbable material such as polyglycolic acid for example. The lumen of the tube contains collagen fibres. The cavities between the collagen fibres are said to enable the ingrowth of nerve tracks. The disadvantage here is the use of a net material for producing the tube, which firstly complicates the production process and secondly introduces additional foreign material into the body of a patient.

US 6,461,629 B1 likewise describes a collagen tube for nerve regeneration. The tube is produced by proceeding from a collagen solution. The collagen solution for producing the tube is injected into a cylindrical device having an inner mandrel for producing a lumen. fibrillogenesis is effected by drying the solution while the fibres can also be aligned by applying a magnetic field.

US20030133967 discloses a process including the technical features of the preamble of claim 1

In view of the known prior art, then, the problem addressed by the present invention is that of providing a very simple, uncomplicated and more particularly universal process for producing medical implants. The implants produced shall be very shape stable and more particularly have a structure which is substantially impervious to liquids, more particularly water and bodily fluids.

This problem is solved according to the present invention by a process for producing a medical hollow body implant, as claimed in claim 1

A liquid-impervious or -impermeable structure is to be understood in the context of the present invention as meaning a structure whose imperviousness to liquids, more particularly water and/or bodily fluids, was evidenced using customary measuring methods known to a person skilled in the art, for example using the Wesolowski method.

An interconnection of the present invention shall also more particularly comprehend an interadherance or lamination, and for the purposes of the present invention it is preferred for the interconnection, more particularly interadherance or lamination, not to be brought about using adhesives such as fibrin adhesives or cyanoacrylate adhesives for example. In other words, it is preferred that the shaped bodies are laminated or laminarly interconnected, preferably without the usage of an adhesive. The interconnection for the purposes of the present invention may be more particularly a chemical and/or physical interconnection. More particularly, the interconnection can be based on adhesive forces or molecular interactions such as for example hydrogen bonds and/or Van der Waals forces between the shaped bodies.

Surprisingly, the use of a solvent, preferably water, is sufficient to interconnect shaped bodies facially, preferably laminatelike, to form a shape-stable hollow body implant having a preferably substantially liquid-impervious structure.

The implant preferably has a structure impervious to water and/or bodily fluids, for example blood, lymph, cerebrospinal fluid, wound exudate or the like.

In a preferred embodiment, shaped bodies having a structure permeable to liquid, more particularly water and/or bodily fluids, are used for the facial interconnection.

Facial interconnection is preferably made using porous and preferably openly porous shaped bodies. The shaped bodies can have pores having a diameter of 10 µm and 500 µm, more particularly 20 µm and 350 µm and preferably 50 µm and 250 µm.

More particularly, shaped bodies can be used for the facial interconnection that have a foam-, sponge- and/or membranelike structure.

In a further embodiment, the facial interconnection is effected using fibrelike or fibre-shaped shaped bodies. It is preferable for the shaped bodies and more particularly at least some of the shaped bodies to have an irregular or randomized fibrous structure.

In a particularly preferred embodiment, the facial interconnection is made using shaped bodies having a nonwovenlike configuration and more particularly using shaped bodies present as a fibrous nonwoven web or as a bonded fibrous nonwoven web fabric. Shaped bodies having a nonwovenlike configuration may, in addition to fibrous structural fractions, also include other structural fractions, for example leaf- or tull-like structural fractions. Shaped bodies configured as a fibrous nonwoven web or as a bonded fibrous nonwoven web fabric are preferably present as fibre webs, more particularly as purely fibre webs.

According to a further embodiment the shaped bodies are selected from the group comprising sponges, foams, patches, membranes, nonwovens or non-woven fabrics, fibrous webs, mats, felts, hybrid structures thereof, and combinations thereof. Hybrid structures are preferably structures exhibiting characteristics of different structures, in particular of structures as mentioned in this or one of the preceding paragraphs. For example, according to the present invention, a hybrid structure may have characteristics of a membrane and a non-woven fabric.

In principle, the hollow body implant can be produced using shaped bodies having different structures, for example as described in the preceding embodiments. Preferably, however, the shaped bodies used for producing the hollow body implant have the same structure.

The shaped bodies provided for producing the hollow body implant may be obtained using moulding processes which are per se familiar to a person skilled in the art. For example, the shaped bodies are obtainable using extrusion, more particularly strand extrusion, fibre extrusion or film/sheet extrusion, spinning, more particularly fibre spinning, pressing, more particularly hot pressing, stamping, more particularly microstamping, embossing, rolling, casting, more particularly injection moulding, or blow moulding, more particularly extrusion blow moulding.

Alternatively, the shaped bodies may be also obtainable by a chemical process, for example via sedimentation, precipitation or crystallization, more particularly from a solution, and subsequent drying.

It may be further conceivable according to the present invention for some of the shaped bodies used for producing the hollow body implant to be obtained by a moulding process and for the other shaped bodies to be obtained by a chemical process.

In a further advantageous embodiment, shaped bodies are facially interconnected which are not obtained via drying processes specific to a porous structure. The facial interconnection is particularly preferably effected using lyophilized (freeze-dried) shaped bodies. Lyophilisation may be envisioned to provide shaped bodies for the facial interconnection which are porous and more particularly fibre shaped and preferably nonwovenlike. Lyophilized shaped bodies are preferably obtained by subjecting liquid dispersions, solutions and/or suspensions, preferably aqueous dispersions, aqueous solutions and/or aqueous suspensions, containing one or more shaped body materials to lyophilisation (freeze drying).

In a further preferred embodiment, sheetlike and preferably flat shaped bodies are used for producing the hollow body implant. It is preferable, in other words, for the shaped bodies - more particularly at least some of the shaped bodies - to be flat bodies or sheet bodies. Suitable shaped bodies are selectable for example from the group comprising individual layers or plies, discs, strips, plates, foils, films, sheets, membranes, gels, more particularly hydrogels, foams, sponges, bands, tapes, ligaments and combinations thereof.

In a particularly preferred embodiment, the sheetlike interconnection is effected using shaped bodies present in the form of individual plies or layers and more particularly in the form of plates. It is more particularly advantageous therein to interconnect shaped bodies configured as nonwovenlike individual plies or layers and more particularly nonwovenlike plates.

A further embodiment of the process for producing the hollow body implant utilizes shaped bodies particularly able to perform a reinforcing function in the final hollow body implant. Suitable shaped bodies can have a membranelike configuration and are preferably recovered from natural membranes, for example the pericardial membrane.

In one possible embodiment, the shaped bodies and more particularly at least some of the shaped bodies are present as particles or particulate shaped bodies, for example as powder, granules or the like.

In a further embodiment, the shaped bodies are dry, dried and/or swollen. For example, the shaped bodies and more particularly at least some of the shaped bodies can be swollen by means of water or an aqueous medium such as an aqueous solution, more particularly aqueous buffer solution, for example.

According to the present invention, it is further possible in principle for the shaped bodies and more particularly at least some of the shaped bodies to be individual fibres or threads.

The shaped bodies may in principle have any desired geometries, sizes and/or thicknesses. More particularly, the shaped bodies can have a spherical, spheroidal, circle-shaped and/or oval-shaped configuration and/or have a polygonal, for example a triangular, quadrangular, more particular square and/or rectangular, pentagonal and/or hexagonal, shape.

The shaped bodies advantageously include a medically compatible material or a medically compatible mixture of materials, for example a mixture of two, three, four, etc different medically compatible materials, and/or are advantageously made of such a material or of such a mixture of materials.

A medically compatible material for the purposes of the present invention is a material which is generally recognized as safe by physicians and, especially after implantation in the body of a patient, causes no or only medically insignificant damage to health, more particularly tissue.

The medically compatible material and the medically compatible mixture of materials are preferably a polymer. The polymer may be more particularly a copolymer, such as for example a random copolymer and/or block copolymer.

A copolymer for the purposes of the present invention is a polymer composed of at least two different monomeric units. Accordingly, the shaped bodies envisioned according to the present invention and more particularly at least some of the shaped bodies may also include and/or be made of a ter- or tetrapolymer.

The medically compatible shaped body material is preferably of xenogenic and more particularly porcine, bovine and/or equine origin. The use of bovine and/or equine materials for producing the shaped bodies is particularly preferred.

In principle, the shaped bodies and, more particularly at least some of the shaped bodies may include or be made of a recombinantly produced material.

In a preferred embodiment, the shaped bodies and more particularly at least some of the shaped bodies include a so-called biopolymer, i.e. a naturally occurring polymer, or a polymer recovered or isolated from natural sources, more particularly animal tissues, and optionally further processed, and/or a synthetic biopolymer. More particularly, the shaped bodies and more particularly at least some of the shaped bodies may be made of a biopolymer or a synthetic, i.e. manufactured, biopolymer.

Preferably, the shaped bodies and more particularly at least some of the shaped bodies include a medically compatible material or are preferably made of a medically compatible material which is selected from the group comprising peptides, oligopeptides, polypeptides such as for example polylysine, polyaminoacids, more particularly synthetic polyaminoacids, proteins, more particularly extracellular proteins or connective tissue proteins, more preferably fibre-shaped or fibrelike proteins, polysaccharides, salts thereof and combinations thereof. Suitable polysaccharides are more particularly selected from the group comprising oxidized polysaccharides, for example saccharides bearing aldehyde groups, alkylcelluloses, hydroxyalkylcelluloses, carboxyalkylcelluloses, amino-bearing polysaccharides, mucopolysaccharides or glycosaminoglycans, salts thereof and mixtures thereof. The peptides, oligopeptides, polypeptides and polyamino acids can be constructed of one single type of amino acid and/or from different amino acids.

The shaped bodies and more particularly at least some of the shaped bodies preferably include or are made of a medically compatible material selected from the group comprising collagen, gelatin, elastin, fibronectin, reticulin, albumin, starch, aldehydic starch, amylose, amylopectin, dextran, dextran aldehyde, cellulose, oxidized cellulose, methylcellulose, hydroxymethylcellulose, hydroxyethylcellulose, hydroxyethylmethyl-cellulose, hydroxypropylmethylcellulose, hydroxybutylmethylcellulose, carboxymethylcellulose, chitin, chitosan, hyaluronic acid, chondroitin 4-sulphate, chondroitin 6-sulphate, dermatan sulphate, keratan sulphate, heparin, heparan sulphate, derivatives thereof, salts thereof and mixtures thereof.

In a further embodiment, the shaped bodies and more particularly at least some of the shaped bodies include or are made of a synthetic polymer preferably selected from the group comprising polyvinyl alcohol, polyvinyl acetate, polyethylene glycol, polyvinylpyrrolidone, poly-ethyleneimine, polyhydroxyalkanoates, poly-ε-caprolactone, poly-trimethylene carbonate, poly-para-dioxanone, copolymers thereof and mixtures, in particular blends, thereof. Preferred polyhydroxyalkanoates may be selected from the group comprising polyglycolide, polylactide, poly-3-hydroxybutyrate, poly-4-hydroxybutyrate, copolymers thereof and mixtures, in particular blends, thereof.

In a particularly preferred embodiment the shaped bodies and more particularly at least some of the shaped bodies include collagen or the shaped bodies and more particularly at least some of the shaped bodies are made of collagen. The collagen is preferably a fibre-forming or fibrillary collagen. Preference is given to collagen of type I, II, III, IV, V, VI, and/or XI. The collagen may be present as native insoluble collagen in particular. A native insoluble collagen for the purposes of the present invention is a collagen which in a chemically unmodified state is insoluble in an aqueous alkaline solution, an aqueous acidic solution or some other aqueous inorganic solution of a salt.

In principle, the shaped bodies may include or may be made of different materials, in particular mixtures of materials. However, it can be preferable according to the present invention, particularly from process-engineering and economic viewpoints, for the shaped bodies each to include and/or be made of the same material or the same mixture of materials. With regard to advantageous materials and mixtures of materials, reference is made to the materials and mixtures of the materials described in the preceding embodiments.

In addition to the medically compatible materials described in the preceding embodiments, the shaped bodies may additionally also contain additives, for example pharmaceutically active substances, salts or the like. With regard to suitable additives reference is made to the description hereinbelow, in particular in connection with the solvent contemplated according to the present invention.

In a further embodiment, shaped bodies used for the facial interconnection have surfaces with a partial or complete coating. Preferably, only one side and more preferably only one facial side of the shaped bodies is coated. Useful coating materials include for example the abovemen-tioned biocompatible materials, of which collagen, gelatin, salts thereof or mixtures thereof are particularly preferred.

The solvent contemplated according to the present invention may be a single solvent or alternatively a solvent mixture.

In a particularly preferred embodiment, the facial interconnection of the shaped bodies is effected using water as solvent. Surprisingly, water turns out to be completely sufficient as interconnecting agent to interconnect the shaped bodies facially to form a shape-stable, more particularly tension- and compression-resistant, hollow body implant which moreover preferably has a substantially, preferably completely, liquid-impervious implant structure.

Alternatively or additionally, organic solvents, preferably hydrophilic solvents, can be used for the facial interconnection of the shaped bodies. A hydrophilic solvent for the purposes of the present invention is an organic solvent which is soluble in water, preferably in any proportion. Thus, an organic solvent which is only partially soluble in water may be principally also encompassed by the term "hydrophilic solvent" as used according to the present invention. Polar protic and/or polar aprotic solvents are preferred in principle. Alcohols, more particularly low molecular weight, preferably water-soluble, alcohols are particularly preferred. The alcohols in question can also be in particular diols and/or triols. Preferred solvents further include ketones, more particularly low molecular weight or short-chain ketones.

Preference is given to using a solvent selected from the group comprising water, methanol, ethanol, propanol, isopropanol, acetone, DMSO and mixtures thereof.

It is particularly advantageous for the solvent contemplated according to the present invention to be free of additives or auxiliaries, more particularly free of adhesive compounds. It may be preferable, in other words, for the shaped bodies to be facially interconnected using a pure solvent, preferably using pure water. The solvent used for this can be in its customary degrees of purity. Alternatively, a pure solvent mixture, or a solvent mixture which is free of adhesives, can also be used for facially interconnecting the shaped bodies.

It may further be envisaged according to the present invention for the solvent to be used for the facial interconnection of the shaped bodies in the form of a dispersion, solution or suspension, more particularly aqueous dispersion, aqueous solution or aqueous suspension.

It may be particularly reasonable from medical viewpoints to endow the hollow body implant with particular properties. For this, it is particularly advantageous to add appropriate additives to the solvent. Preferably, the solvent contains additives selected from the group comprising inorganic salts, more particularly alkali metal and/or alkaline earth metal halides, organic salts, more particularly fatty acid salts, medical/medicinal/- pharmaceutical substances, more particularly antimicrobial substances, preferably antibiotics such as for example gentamycin, polyhexamethyl-enebiguanide, triclosan, copper, silver, gold or salts thereof, disinfectants, anti-inflammatory substances, wound healing promoter substances, analgesic substances, odour control substances, cellular growth factors, cellular differentiation factors, cellular recruitment factors, cellular adhesion factors, cell binding sequences, plasticizers such as for example glycerol and combinations thereof. Additizing the solvent with inorganic salts is a way to increase the conductivity of the implant for example. This is particularly advantageous for example to perform anastomoses or vessel sealing using the thermal fusion technique (TFT) in particular. Additizing the solvent with water-soluble salts has the further advantage that these salts can be dissolved out of the implant structure in the body of a patient. This creates holes or pores in the implant structure to speed the tissue regeneration and/or remodelling by the ingrowth of endogenous cells for example. Alternatively, these salts can also be specifically leached and/or dissolved out of the implant prior to implantation.

Alternatively, the hollow body implant can also be additized after it has been produced, more particularly after the facial interconnection of the shaped bodies. To this end, the implant can be endowed or finished with the desired additives as part of a post-treatment for example. With regard to suitable additives, reference is made to the additives described in the preceding embodiment.

The shaped bodies to be facially interconnected are moistened or wetted with the solvent. To moisten the shaped bodies with the solvent, they can be brushed, sprayed, overpoured with the solvent and/or dipped or immersed into the solvent. The moistening of the shaped bodies can optionally also be repeated. Preferably, the shaped bodies are sprayed with the solvent for facial interconnection.

To produce the hollow body implant, preferably a tubular or tube-shaped implant, the shaped bodies are facially interconnected on a carrier responsible for forming a cavity. To this end, the shaped bodies are preferably mounted on the carrier in a positively locking fashion (form-fit fashion), or the carrier is preferably ensheathed or overwrapped by the shaped bodies in a positively locking fashion (form-fit fashion). The carrier used can be a pin, a mandrel, a mould core, tube, cylinder or the like. The shape or geometry, more particularly the cross-section, of the carrier determines the shape or geometry, more particularly the cross-section, of the hollow body of the implant. The carrier may have for example a circularly round, oval-shaped or polygonal, more particularly triangular, quadrangular, more particularly square or rectangular, pentagonal, hexagonal or star-shaped, cross-section. A circularly round cross-section of the carrier is preferred. The carrier more preferably has a tubular or tube-shaped or conical (cylindrical) configuration.

The shaped bodies are preferably laid or stacked onto each other on the carrier. Alternatively, the shaped bodies can also be needled together on the carrier. It is particularly preferable for solvent-moistened shaped bodies configured as flat or sheet bodies, more particularly as individual plies or layers, to be laminated on top of each other on the carrier.

In an advanced embodiment, the shaped bodies are placed or stacked onto each other on the carrier responsible for forming a cavity such that mutually opposite ends, more particularly transversal side ends, of the shaped bodies touch, abuttingly. The end of a shaped body to be mounted on the carrier is to be mounted abuttingly in respect of the start of a shaped body previously mounted on the carrier. Interface inhomogeneities in the implant structure can be avoided in this way to particular advantage.

It is particularly preferable to perform a drying operation, more particularly a warm drying operation, to achieve a durable interconnection of the shaped bodies. Preferably, drying is carried out with at least partial and preferably complete elimination or removal of layer or phase boundaries between the shaped bodies. The drying temperature can be particularly advantageously adapted to the thermal sensitivity of the shaped body materials used. Any drying operation is preferably carried out in a temperature range between 20°C and 80°C, more particularly 25°C and 45°C and preferably 30°C and 40°C. Alternatively, the shaped bodies can also be dried by lyophilisation (freeze drying). The facially interconnected shaped bodies are preferably dried on a carrier responsible for forming a cavity. It is a particular advantage that a single drying step is completely sufficient for producing the laminate.

In a particularly preferred embodiment, the shaped bodies are facially interconnected by the solvent-moistened shaped bodies being laid or stacked onto each other and then dried. Multiple interconnecting and more particularly drying steps can be avoided in this way.

To establish adequate contact between the shaped bodies for facial interconnection, the shaped bodies can be subjected to a force, preferably a pressing force, before and/or during any drying step in particular.

In principle, any number of shaped bodies can be used to produce the medical hollow body implant. Preferably the number of shaped bodies facially interconnected is in the range from 2 to 15, more particularly in the range from 2 to 10 and more preferably in the range from 2 to 5. The number of shaped bodies used for producing the implant is a particularly advantageous way to improve/enhance the shape stability and more particularly imperviousness of the hollow body implant. However, a particular advantage of the present invention resides in the fact that liquid-impervious hollow body implants are obtainable in the facial interconnection of just two shaped bodies.

Although the implants of the present invention are in and of themselves already sufficiently shape-stable and more particularly liquid-impervious, the present invention does make it possible for the hollow body implant to be crosslinked in a subsequent treatment step for example. Thermal, radiation-induced and/or chemical methods of crosslinking can be used for this. The crosslinking of the hollow body implant can take place more particularly in the gas phase and/or in a solution. Preferably, the hollow body implant is crosslinked using chemical crosslinking agents preferably selected from the group comprising formaldehyde, dialdehydes, more particularly glutaraldehyde, carbodiimides, diisocyanates, for example hexamethylene diisocyanate, bifunctional succinimides and combinations thereof.

In an advanced embodiment, the implant is crosslinked in the presence of a plasticizer, for example glycerol.

The present invention further provides a medical hollow body implant, more particularly tubular or tube-shaped implant, obtained or obtainable via or by means of a facial, two-dimensional or sheetlike interconnection of shaped bodies (formed bodies) which are preferably present in the form of individual plies or layers, using a solvent, preferably to construct or form an essentially liquid-impervious, preferably completely liquid-impervious, implant structure.

The hollow body implant is particularly advantageous in having high dimensional stability, preferably breaking strength and more particularly compressive resistance.

The hollow body implant preferably has a structure impervious to water and/or bodily fluids.

Preferably, the interconnected shaped body faces, more particularly at least some of the interconnected shaped body faces, do not form any layer or phase boundaries in the final implant. In other words, it may be preferable according to the present invention for the hollow body implant not to have any layer or phase boundaries originating in the facial interconnection of the shaped bodies. More particularly, the implant can be completely free of layer or phase boundaries.

In a further advantageous embodiment, the hollow body implant is free of securing or fixing means, more particularly free of adhesives, stitches, staples or the like.

It is further preferable for the hollow body implant to have a linear breaking force or strength of above 2 N, more particularly above 5 N and preferably above 10 N.

Furthermore, the hollow body implant may have a linear breaking force or strength from 2 to 400 N, in particular from 5 to 300 N and preferably from 10 to 200 N.

The hollow body implant may further have a radial breaking force or strength of above 2 N, more particularly above 5 N and preferably above 10 N.

Furthermore, the hollow body implant may have a radial breaking force or strength from 2 to 400 N, in particular from 5 to 300 N and preferably from 10 to 200 N.

More particularly, the hollow body implant may have a thread pull-out force or strength of above 2 N, more particularly above 5 N and preferably above 10 N.

Furthermore, the hollow body implant may have a thread pull-out force or strength from 2 to 400 N, in particular from 5 to 300 N and preferably from 10 to 200 N.

In a preferred embodiment, the hollow body implant is configured as a tube, as a cone or as a ring. A ring-shaped implant may be obtainable for example by trimming a tube-shaped implant obtained according to the present invention.

As mentioned more than once, the hollow body implant is preferably a tubular or tube-shaped (cylindrical) implant. Preferably, the hollow body implant is configured as a tube based on proteins and/or salts thereof, more particularly extracellular proteins or connective tissue proteins and/or salts thereof, preferably of collagen, gelatin and/or salts thereof.

In a further embodiment, the hollow body implant is sterilizable, preferably without the advantageous properties of the implant being impaired hereby. It is therefore preferable according to the present invention if the implant is in a sterilized state. The hollow body implant may be in a sterilized state following γ-sterilization and/or ethylene oxide sterilization for example.

The medical hollow body implant is preferably used as a surgical implant. The implant of the present invention is preferably provided for reconstruction, augmentation, sealing and/or fusion of human and/or animal tissues, more particularly hollow organs and/or vessels, preferably blood vessels. To carry out tissue fusions, more particularly anastomoses, it is preferable to use the implant.

The implant of the present invention may further also be useful as a tissue guide reel, more particularly for nerve tissue or nerve tracks.

With respect to further advantages and features of the hollow body implant of the present invention, reference is made in its entirety to the advantages and features of the process according to the present invention.

The advantages of the invention will now be summarised once more:
The present invention is based on the surprising realization that the interconnecting force of a solvent, pure water in particular, is sufficient to facially interconnect individual shaped bodies to form shape-stable hollow body implants. To achieve shape stability which is adequate from medical viewpoints, it is especially not necessary to subject the implants to a subsequent crosslinking step. Nor is the use of adhesives or adhesive additives necessary to obtain shape-stable implants. Tedious and laborious polymerization/curing periods can be avoided as a result. Nor are fixing aids such as for example sutures, staplers or the like needed to carry out the process of the present invention. This is particularly advantageous in reducing the input of foreign material in the body of a patient. Altogether, the process of the present invention represents a significantly simplified and more particularly more economical way to produce patient-compatible implants compared with the production processes known from the prior art.

A further advantage of the process according to the present invention concerns its universal character. The process according to the present invention is able to provide in principle hollow body implants having a very wide variety of diameters, shapes, geometries and/or thicknesses.

As mentioned more than once, the implants of the present invention are advantageously notable for excellent dimensional stability. This excellent dimensional stability manifests particularly in a breaking strength and particularly compressive strength which are optimal for medical purposes. The implants are thereby readily able to withstand the forces typically occurring in the body of a patient. A further advantage concerns the preferably liquid-impervious structure of the implant. A diversity of possible medical uses arise from it. The implants of the present invention are particularly useful for carrying out tissue fusions, more particularly anastomoses. They are also usable as a cell tissue guide rail, for example for nerve tissue.

Further features, details and advantages of the present invention will be apparent from the following description of preferred embodiments with reference to examples combined with the dependent claims. Features can each be actualized singly or in combination with each or one another. The examples merely serve to illustrate the invention and are not in any way to be understood as limiting.

### Example 1

### Producing a collagen tube from two collagen nonwovens

Two collagen nonwovens, each having a basis weight of about 125 g/m² and a size of 40 x 16 cm (640 cm²), were moistened with water using a spray applicator. Then, a cylindrical carrier form (25 mm outer diameter and 40 cm length) was ensheathed with one of the two moistened collagen nonwovens in a positively locking fashion. This was followed by a second overwrapping of the carrier form with the other moistened collagen nonwoven (covering ply). To avoid interface inhomogeneities, the end of the second collagen nonwoven was laminated abuttingly onto the beginning of the first wrap. The wrapped collagen nonwovens were then dried at 37°C for 24 hours and subsequently removed from the carrier form. The collagen tube obtained in a length of 40 cm had a basis weight of about 250 g/m².

### Example 2

### Collagen tube from four collagen nonwovens with subsequent crosslinking

Four collagen nonwovens having a basis weight of about 125 g/m² and a size of 40 x 32 cm (1280 cm²) were moistened with water using a spray applicator. Then, a cylindrical carrier form (25 mm outer diameter and 40 cm length) was ensheathed with one of the moistened collagen nonwovens in a positively locking manner. This was followed by three further overwrappings of the carrier form with the other moistened collagen nonwovens (covering plies). To avoid interface inhomogeneities, the end of the most recently applied collagen nonwoven was laminated abuttingly onto the beginning of the previously wrapped collagen non-woven. The wrapped collagen nonwovens were subsequently dried at 37°C for 24 hours and then removed from the carrier form. The collagen tube obtained in a length of 40 cm had a basis weight of about 500 g/m². To enhance its stability, the collagen tube was dipped for 6 hours into a crosslinking solution of 75 g of isopropanol, 8.04 g of deionized water, 16.87 g of glycerol and 90.82 µl of hexamethylene diisocyanate (HMDI). Subsequently, unbound hexamethylene diisocyanate was rinsed off and the crosslinked collagen tube was again dried at 37 °C.

### Example 3

### Producing a collagen tube from three thin collagen nonwovens with subsequent crosslinking

Three thin collagen nonwovens having a basis weight of about 85 g/m² and a size of 40 x 24 cm (960 cm²) were moistened with water using a spray applicator. Thereafter, a cylindrically shaped carrier form (25 mm outer diameter and 40 cm length) was overwrapped with one of the moistened collagen nonwovens in a positively locking fashion. This was followed by two further overwrappings with the other two moistened collagen nonwovens (covering plies). To avoid interface inhomogeneities, the end of the most recently wrapped collagen web was laminated abuttingly onto the beginning of the previously wrapped collagen non-woven. The wrapped collagen nonwovens were then dried at 37°C for 24 hours. After drying, the wrapped collagen nonwovens were removed from the carrier form to obtain a collagen tube 40 cm in length and having a basis weight of about 255 g/m². To enhance its stability, the collagen tube was dipped for 6 hours into a crosslinking solution of 75 g of isopropanol, 8.04 g of deionized water, 16.87 g of glycerol and 90.82 µl of hexamethylene diisocyanate (HMDI). Unbound hexamethylene diisocyanate was subsequently rinsed off and the collagen tube again dried at 37 °C.

The specimens produced in Examples 1 to 3 were each subjected to a γ sterilization process and an ethylene oxide sterilization process after drying. In this case, the specimens did not show changes in the sterilized state, especially no disadvantageous changes.

### 4. Characterizing the collagen tubes produced in Examples 1 to 3

### 4.1 Determining the linear breaking force

The collagen tubes produced in Examples 1 to 3 were each cut to excise 10 mm wide and 50 mm long strips as sample materials. The sample strips were clamped in the dry state, or after swelling in aqueous buffer solution (pH 7.4), in a tensile tester to determine the breaking force of the strips. The results obtained are shown in Tables 1 and 2.

### 4.2 Determining the radial breaking force

The collagen tubes produced in Examples 1 to 3 were cut to excise rings 10 mm in width. The rings were threaded in the dry state, or after swelling in an aqueous buffer solution (pH 7.4) onto the holders of a tensile tester, in order to measure the breaking force. The results are likewise reproduced in Tables 1 and 2.

### 4.3 Determining the thread pull-out force

The collagen tubes produced in Examples 1 to 3 were cut to excise sample pieces measuring 4 x 2 cm. The sample pieces in the dry state of after swelling in an aqueous buffer solution (pH 7.4), were pierced from above at 1 cm from the lateral edge with a monofil suture of USP 2-0 size and fixed into the upper jaw of a tensile tester. The lower end of the sample pieces was fixed in the lower jaw. The subsequent measurement was used to determine the maximum force until complete pull-out of the thread from the sample body. The results are likewise reproduced in Tables 1 and 2.

**Table 1**

| Sample | Linear breaking force dry | Radial breaking force dry | Thread pull-out force dry |
|---|---|---|---|
| Example 1 | 9.5 N | 12.6 N | 5.4 N |
| Example 2 | 22.1 N | 28.1 N | 23.4 N |
| Example 3 | 12.7 N | 14.3 N | 8.9 N |

**Table 2**

| Sample | Linear breaking force after swelling | Radial breaking force after swelling | Thread pull-out force after swelling |
|---|---|---|---|
| Example 1 | 3.9 N | 8.9 N | 3.6 N |
| Example 2 | 10.1 N | 13.7 N | 11.3 N |
| Example 3 | 4.4 N | 9.3 N | 4.9 N |

### 4.4 Determining imperviousness under static pressure

The collagen tubes produced in Examples 1 to 3 were each cut to tubes having a shorter length. The latter were used as test specimens and sealed on one side, filled with deionized water and finally exposed to compressed air at 120 mmHg for 30 minutes. Imperviousness was checked thereafter. The result is shown in Table 3.

### 4.5 Determining imperviousness under dynamic pressure

The collagen tubes produced in Examples 1 to 3 were each cut into tubes of smaller length. The latter were used as test specimens and fitted both sides with adaptors for the imperviousness tester and placed into a closed container filled with deionized water. The adaptors were coupled to a pump system and exposed to a pulsating pressure of 180/90 mmHg at a frequency of 70 per minute for 60 minutes. This was followed by an inspection for imperviousness through displaced water at the overflow. The results are likewise shown in Table 3.

### 4.6 Determination of Wesolowski imperviousness

The collagen tubes produced in Examples 1 to 3 were trimmed to sample pieces measuring 2.5 x 2.5 cm, which were inserted into a clamping device. After activation of the system, the sample pieces were each loaded with a pressure of a water column 166 cm in length (corresponds to 122 mmHg) on an area of 1 cm². Imperviousness was determined by the amount of water permeating through the sample during one hour. The results are likewise shown in Table 3.

**Table 3**

| Sample | Static imperviousness | Dynamic imperviousness | Wesolowski |
|---|---|---|---|
| Example1 | impervious | impervious | impervious |
| Example2 | impervious | impervious | impervious |
| Example3 | impervious | impervious | impervious |

## Claims

1. Process for producing a medical hollow body implant, more particularly a tubular or tube-shaped implant, wherein shaped bodies are facially interconnected by means of a solvent, the shaped bodies to be facially interconnection are moistened with the solvent, are facially interconnected on a carrier responsible for forming a cavity, **characterized in that** the shaped bodies are placed onto each other on the carrier, wherein the end of a shaped body to be mounted on the carrier is mounted abuttingly in respect of the start of a shaped body previously mounted on the carrier.

2. Process according to Claim 1, **characterized in that** shaped bodies used for the facial interconnection have a porous, more particularly an openly porous, structure, the facial inter¬connection is in particular effected using fibrelike or fibre-shaped shaped bodies, preferably using nonwovenlike shaped bodies.

3. Process according to claim 1 or 2, **characterized in that** the facial interconnection is effected using lyophilized shaped bodies.

4. Process according to any preceding claim, **characterized in that** the facial interconnection is effected using shaped bodies in the form of sheet bodies and more particularly in the form of individual plies or layers.

5. Process according to any preceding claim, **characterized in that** the shaped bodies include or are made of a biopolymer selected from the group comprising peptides, oligopeptides, polypeptides, polyamino acids, proteins, more particularly extracellular proteins or connective tissue proteins, polysaccharides, more particularly oxidized poly¬saccharides, mucopolysaccharides or glycosaminoglycans, salts thereof and combinations thereof.

6. Process according to any preceding claim, **characterized in that** water and/or a hydrophilic solvent, more particularly a water-soluble alcohol, is used as solvent.

7. Process according to any preceding claim, **characterized in that** the facial interconnection is effected using purely solvent or purely solvent mixture.

8. Process according to any one of claims 1 to 6, **characterized in that** the solvent is used in the form of a dispersion, solution or suspension.

9. Process according to any one of claims 1 to 6 or 8, **characterized in that** a solvent used for the facial interconnection of the shaped bodies contains additives, preferably selected from the group comprising inorganic salts, more particularly alkali metal and/or alkaline earth metal halides, organic salts, more particularly fatty acid salts, medical or pharmaceutical substances, more particularly antimicrobial substances, preferably antibiotics, disinfectants, anti-inflammatory substances, wound healing promoter substances, analgesic substances, odour control substances, cellular growth factors, cellular differentiation factors, cellular recruitment factors and/or cellular adhesion factors, plasticizers and combinations thereof.

10. Process according to any preceding claim, **characterized in that** a tubular or tube-shaped implant is produced by facially interconnecting the shaped bodies on a tubular or tube-shaped or conically shaped carrier.

11. Process according to Claim 10, **characterized in that** solvent-moistened shaped bodies which are preferably configured as individual plies or layers are laid or stacked onto each other, more particularly laminated on top of each other, on the carrier.

12. Process according to any preceding claim, **characterized in that** the shaped bodies are facially interconnected by drying, more particularly in a temperature range between 20°C and 80°C, more particularly 25°C and 45°C and preferably 30°C and 40°C.

13. Process according to any preceding claim, **characterized in that** the hollow body implant is crosslinked, for example in a subsequent treatment step and preferably in the presence of a plasticizer.

## Patentansprüche

1. Verfahren zur Herstellung eines medizinischen Hohlkörperimplantats, insbesondere eines tubulären bzw. rohrförmigen Implantats, bei welchem Formkörper mittels eines Lösungsmittels flächig miteinander verbunden werden, die flächig miteinander zu verbindenden Formkörper mit dem Lösungsmittel befeuchtet werden und auf einem für die Ausbildung eines Hohlraumes verantwortlichen Träger flächig miteinander verbunden werden, **dadurch gekennzeichnet, dass** die Formkörper auf dem Träger übereinander gelegt werden, wobei das Ende eines auf den Träger anzubringenden Formkörpers auf Stoß auf den Anfang eines zuvor auf den Träger angebrachten Formkörpers aufgebracht wird.

2. Verfahren nach Anspruch 1, **dadurch gekennzeichnet, dass** zur flächigen Verbindung verwendete Formkörper eine poröse, insbesondere offenporöse, Struktur aufweisen und die flächige Verbindung insbesondere unter Verwendung von faserartigen bzw. faserförmigen Formkörpern, vorzugsweise mit vliesartigen Formkörpern, durchgeführt wird.

3. Verfahren nach Anspruch 1 oder 2, **dadurch gekennzeichnet, dass** die flächige Verbindung mit lyophilisierten Formkörpern vorgenommen wird.

4. Verfahren nach einem der vorhergehenden Ansprüche, **dadurch gekennzeichnet, dass** die flächige Verbindung mit in Form von Flächengebilden, insbesondere mit in Form von einzelnen Lagen bzw. Schichten, vorliegenden Formkörpern durchgeführt wird.

5. Verfahren nach einem der vorhergehenden Ansprüche, **dadurch gekennzeichnet, dass** die Formkörper ein Biopolymer aufweisen bzw. aus einem Biopolymer hergestellt sind, welches ausgewählt ist aus der Gruppe umfassend Peptide, Oligopeptide, Polypeptide, Polyaminosäuren, Proteine, insbesondere extrazelluläre Proteine bzw. Bindegewebsproteine, Polysaccharide, insbesondere oxidierte Polysaccharide, Mucopolysaccharide bzw. Glycosaminoglykane, Salze davon und Kombinationen davon.

6. Verfahren nach einem der vorhergehenden Ansprüche, **dadurch gekennzeichnet, dass** Wasser und/oder ein hydrophiles Lösungsmittel, insbesondere ein wasserlöslicher Alkohol, als Lösungsmittel verwendet wird.

7. Verfahren nach einem der vorhergehenden Ansprüche, **dadurch gekennzeichnet, dass** die flächige Verbindung mit einem reinen Lösungsmittel oder einem reinen Lösungsmittelgemisch vorgenommen wird.

8. Verfahren nach einem der Ansprüche 1 bis 6, **dadurch gekennzeichnet, dass** das Lösungsmittel in Form einer Dispersion, Lösung oder Suspension verwendet wird.

9. Verfahren nach einem der Ansprüche 1 bis 6 oder 8, **dadurch gekennzeichnet, dass** zur flächigen Verbindung der Formkörper ein Lösungsmittel verwendet wird, welches Additive enthält, vorzugsweise ausgewählt aus der Gruppe umfassend anorganische Salze, insbesondere Alkalimetall- und/oder Erdalkalimetallhalogenide, organische Salze, insbesondere Fettsäuresalze, medizinische bzw. pharmazeutische Substanzen, insbesondere antimikrobielle Substanzen, bevorzugt Antibiotika, Desinfektionsmittel, entzündungshemmende Substanzen, wundheilungsfördernde Substanzen, schmerzlindernde Substanzen, geruchsbekämpfende Substanzen, zelluläre Wachstumsfaktoren, zelluläre Differenzierungsfaktoren, zelluläre Rekrutierungsfaktoren und/oder zelluläre Adhäsionsfaktoren, Weichmacher und Kombinationen davon.

10. Verfahren nach einem der vorhergehenden Ansprüche, **dadurch gekennzeichnet, dass** ein tubuläres bzw. rohrförmiges Implantat durch flächiges Verbinden der Formkörper miteinander auf einem tubulären oder rohrförmigen oder konisch geformten Träger ausgebildet wird.

11. Verfahren nach Anspruch 10, **dadurch gekennzeichnet, dass** mit dem Lösungsmittel befeuchtete Formkörper, welche vorzugsweise als einzelne Lagen bzw. Schichten ausgebildet sind, auf dem Träger übereinander gelegt bzw. übereinander gestapelt, insbesondere übereinander laminiert, werden.

12. Verfahren nach einem der vorhergehenden Ansprüche, **dadurch gekennzeichnet, dass** zur flächigen Verbindung der Formkörper eine Trocknung, insbesondere in einem Temperaturbereich zwischen 20 °C und 80 °C, insbesondere 25 °C und 45 °C, bevorzugt 30 °C und 40 °C, durchgeführt wird.

13. Verfahren nach einem der vorhergehenden Ansprüche, **dadurch gekennzeichnet, dass** das Hohlkörperimplantat, beispielsweise in einem nachträglichen Behandlungsschritt und vorzugsweise in Gegenwart eines Weichmachers, vernetzt wird.

## Revendications

1. Procédé de fabrication d'un implant médical à corps creux, plus particulièrement un implant tubulaire ou en forme de tube, dans lequel des corps façonnés sont interconnectés facialement au moyen d'un solvant, les corps façonnés à interconnecter facialement sont humidifiés avec le solvant, sont interconnectés facialement sur un support responsable de la formation d'une cavité, **caractérisé en ce que** les corps façonnés sont placés les uns sur les autres sur le support, l'extrémité d'un corps façonné à monter sur le support étant montée de façon à se trouver contre le début d'un corps façonné monté précédemment sur le support.

2. Procédé selon la revendication 1, **caractérisé en ce que** les corps façonnés utilisés pour l'interconnexion faciale ont une structure poreuse, plus particulièrement une structure poreuse ouverte, l'interconnexion faciale étant effectuée en particulier en utilisant des corps façonnés semblables à des fibres ou en forme de fibres, préférablement en utilisant des corps façonnés semblables à des non-tissés.

3. Procédé selon la revendication 1 ou 2, **caractérisé en ce que** l'interconnexion faciale est effectuée en utilisant des corps façonnés lyophilisés.

4. Procédé selon l'une quelconque des revendications précédentes, **caractérisé en ce que** l'interconnexion faciale est effectuée en utilisant des corps façonnés sous forme de corps de type feuille et plus particulièrement sous forme de plis ou de couches individuelles.

5. Procédé selon l'une quelconque des revendications précédentes, **caractérisé en ce que** les corps façonnés contiennent ou se composent d'un biopolymère sélectionné dans le groupe comprenant des peptides, des oligopeptides, des polypeptides, des acides polyaminés, des protéines, plus particulièrement des protéines extracellulaires ou des protéines de tissu conjonctif, des polysaccharides, plus particulièrement des polysaccharides oxydés, des mucopolysaccharides ou des glycosaminoglycanes, des sels de ceux-ci et des combinaisons de ceux-ci.

6. Procédé selon l'une quelconque des revendications précédentes, **caractérisé en ce que** de l'eau et/ou un solvant hydrophile, plus particulièrement un alcool hydrosoluble, est/sont utilisé(s) comme solvant.

7. Procédé selon l'une quelconque des revendications précédentes, **caractérisé en ce que** l'interconnexion faciale est effectuée en utilisant un solvant pur ou un mélange de solvants purs.

8. Procédé selon l'une quelconque des revendications 1 à 6, **caractérisé en ce que** le solvant est utilisé sous la forme d'une dispersion, d'une solution ou d'une suspension.

9. Procédé selon l'une quelconque des revendications 1 à 6 ou 8, **caractérisé en ce qu'**un solvant utilisé pour l'interconnexion faciale des corps façonnés contient des additifs, préférablement sélectionnés dans le groupe comprenant des sels inorganiques, plus particulièrement des halogénures de métaux alcalins et/ou de métaux alcalino-terreux, des sels organiques, plus particulièrement des sels d'acides gras, des substances médicales ou pharmaceutiques, plus particulièrement des substances antimicrobiennes, préférablement des antibiotiques, des désinfectants, des substances anti-inflammatoires, des substances favorisant la cicatrisation des plaies, des substances analgésiques, des substances anti-odeur, des facteurs de croissance cellulaire, des facteurs de différenciation cellulaire, des facteurs de recrutement cellulaire et/ou des facteurs d'adhérence cellulaire, des plastifiants et des combinaisons de ceux-ci.

10. Procédé selon l'une quelconque des revendications précédentes, **caractérisé en ce qu'**un implant tubulaire ou en forme de tube est produit par interconnexion faciale des corps façonnés sur un support tubulaire ou en forme de tube ou de forme conique.

11. Procédé selon la revendication 10, **caractérisé en ce que** des corps façonnés humidifiés par un solvant qui sont préférablement configurés sous forme de plis ou de couches individuelles sont disposés ou empilés les uns sur les autres, plus particulièrement disposés par stratification les uns sur les autres, sur le support.

12. Procédé selon l'une quelconque des revendications précédentes, **caractérisé en ce que** les corps façonnés sont interconnectés facialement par séchage, plus particulièrement dans une plage de températures de 20 °C à 80 °C, plus particulièrement de 25 °C à 45 °C, et préférablement de 30 °C à 40 °C.

13. Procédé selon l'une quelconque des revendications précédentes, **caractérisé en ce que** l'implant à corps creux est réticulé, par exemple dans une étape de traitement ultérieure, et préférablement en présence d'un plastifiant.
